# EUROPEAN PATENT SPECIFICATION

(11) **EP 0 463 024 B1**
(45) Date of publication and mention of the grant of the patent: **01.08.2001**
(21) Application number: 90904399.4
(22) Date of filing: 13.03.1990
(51) Int. Cl.: C12N 15/56, C12N 15/01, C12N 1/19, C12P 21/02, C12P 7/06, A23K 1/08

(54) **DNA CONSTRUCT AND MODIFIED YEAST**
DNA-ZUSAMMENSETZUNG UND MODIFIZIERTE HEFE
CONSTRUCTION D'ADN ET LEVURE MODIFIEE

(30) Priority: 13.03.1989 GB 8905674
(43) Date of publication of application: 02.01.1992
(73) Proprietor: Elsworth Biotechnology Limited, Elsworth, Cambridge CB2 8HY (GB)
(72) Inventor: HARTLEY, Brian, Selby, Cambridge Cambridgeshire CB2 8HY (GB); RAMAKRISHNAN, Sundaram, London SW7 3BQ (GB); KUMAR, Vijay, West Midlands WV3 0LQ (GB)
(74) Representative: Tombling, Adrian George
(86) International application number: GB9000373
(87) International publication number: WO9010703

(56) References cited:
- EP-A- 0 201 239
- EP-A- 0 206 571
- WO-A-87/02984
- GB-A- 2 178 431
- Trends in Biotechnology, Vol. 4, No. 5, May 1986, Elsevier Science Publishers B.V., (Amsterdam, NL), I. RUSSELL: "Will a Recombinant DNA Yeast be able to Solve whey Disposal Problems?", pages 107-108
- Molecular and General Genetics, Vol. 194, 1984, Springer-Verlag, (Berlin, DE), M.E. PENTTILA et al.: "Cloning of Aspergillus Niger Genes in Yeast. Expression of the Gene Coding Aspergillus beta-Glucosidase", pages 494-499

## Description

This invention relates to DNA constructs and to a novel yeast vector containing same. In particular it relates to novel strains of yeast for utilisation of whey.

Whey is a major by-product of cheese and casein manufacture, amounting to 4 kg./kg. of cheese and 14 kg./kg. of casein. World production was about 50 million tonnes in 1981 (F.A.O. Production Yearbook, 35, 234, 1981). It contains 14% of the original milk fat, 22% of the protein, 74% of the ash and 98% of the lactose. The protein has significant value as baby food and in food manufacture, and is increasingly being separated and concentrated for this purpose by ultrafiltration. However, the lactose and salts have lower value and are frequently simply discarded. Without expensive sewage treatment, this can represent a major source of water pollution, so there is strong political and environmental pressure to find alternative uses for whey in general and for whey permeate in particular.

Several traditional avenues for utilisation of whey were reviewed by Wix and Woodbine in 1958 (Dairy Sci. Abstr., 20, 538). Direct use as animal feed is popular where the market is close to the point of production, so that transport costs are minimised. Another 'local' use is as an addition to silage alongside 'starter cultures' of lactic acid bacteria. It allows them to produce acids more quickly and thereby eliminate competition from 'spoilage organisms' that prefer more neutral pH.

Both whey and whey permeate can be fermented aerobically by yeasts such as Kluyveromyces fragilis to yield high levels of single cell protein (SCP) for animal feed. By-products such as ethanol and lactic acid are also formed, but in continuous plants a mixed flora of other yeasts that can utilise these by-products evolves alongside K. fragilis (Moulin et al, Biotech. Bioeng. 22, 1277, 1980). There is concern that filamentous fungi producing aflatoxins may also arise in such plants. Several major milk-producing countries now operate plants converting whey permeate to ethanol. Again Kluyveromyces.species are used, but in anaerobic fermentations. The Carberry plant in Ireland, for example, produces 2.59% v/v of ethanol from 4.7 w/v of lactose in whey permeate (Barry, J.A., Dairy Ind. Intnl., Oct. 1982, p. 19). Because the Kluyveromyces species currently used have low ethanol tolerance, pre-concentration of the lactose is not possible, so fermentation and distillation costs are considerable.

Yeasts such as Saccharomyces cerevisiae are the organisms of choice for ethanol fermentations because they have high ethanol and sugar tolerance and are already widely used and acceptable in human food (bread) and drinks (beer and wine). But with respect to whey utilisation, Saccharomyces cerevisiae is at a distinct disadvantage in that it is unable to utilise lactose directly because it lacks both a lactose uptake system and a β-galactosidase (β-D-galactoside galactohydrolase, E.C.3.2.1.23), (R.C. Dickson, Gene, 10, 347, 1980). However, it can utilise glucose and galactose. Lactose utilisation has been demonstrated using whey hydrolysed with a β-galactosidase, but this entails an expensive pre-treatment stage. Alternatively yeast cells can be co-immobilised with β220-galactosidase from the fungus Aspergillus oryzae (O'Leary et al, Biotech. Bioeng., 19, 1019, 1977; B. Hahn-Hagerdal, Biotech. Bioeng., 27, 914, 1984), but batch cultures of co-immobilised yeast lost activity due to shearing by stirring and budding of cells. This problem can be overcome by using a packed-bed reactor in which the packed yeast cells are unable to grow, but the β-galactosidase activity is stable for only three weeks (W. Hartmeier, Biotechnology, 2. 594, 1984). Moreover such a mixed-culture requires complex management.

There have been several attempts to construct a strain of S. cerevisiae able to utilise lactose. Sreekrishna and Dickson (1985) cloned a 13-kilobase fragment of K. lactis DNA which included the β-galactosidase gene (LAC4) into S. cerevisiae on a yeast replicating plasmid which also confers resistance to the antibiotic G418 in yeast. The β-galactosidase is intracellular, and a membrane-bound lactose permease is also necessary to allow the host to grow on lactose. Fortuitously this permease gene was also cloned and became expressed when integrated at a specific S. cerevisiae locus. However, the transformants grew much more slowly (doubling time 6.7 hr) than K. lactis itself (1.6 hr) and the phenotype proved to be unstable.

Protoplast fusion has been used to construct hybrids between auxotrophic strains of Saccharomyces cerevisiae having a high ethanol tolerance and an auxotrophic strain of lactose-fermenting Kluyveromyces fragilis isolated by ethyl methanesulphonate mutagenesis (Farahnak et al., Applied Environ. Microbiol., 51, 362, 1986). The fusants obtained were reported to be prototrophic and to be capable of assimilating lactose and producing ethanol in excess of 13% (vol/vol). Kluyveromyces fragilis has a membrane-bound lactose permease and it would appear that this allows the fusant strain to utilise its intracellular galactosidase and hence grow on lactose.

In GB-A-2178431 there is described a yeast cell that has been transformed with DNA which confers on said yeast cell the ability to utilise lactose as a carbon source. Such DNA can be derived from Kluyveromyces fragilis, whilst the yeast that is transformed may be of the genus Saccharomyces, e.g. S. cerevisiae or S. uvarum. It would appear that the transformed yeast expresses β-galactosidase but requires also the presence in the transforming DNA of DNA encoding a protein exhibiting lactose permease activity which thus facilitates entry of lactose into the yeast cell. In other words the transformed yeast cell of GB-A-2178431 expresses β-galactosidase intracellularly.

It would be desirable to produce yeasts that can utilise lactose more efficiently.

Accordingly, a first aspect of the present invention provides a yeast capable of secreting a protein expressed thereby, the yeast having been transformed with a DNA construct comprising DNA coding for a β-galactosidase, said DNA being obtainable from a source in which the β-galactosidase is naturally secreted extracelluarly, said DNA being flanked at its 5' end by a yeast leader sequence, whereby the yeast is capable of growth on lactose as the sole C source.

The DNA coding for the β-galactosidase may be flanked at its 3' end by a terminator sequence.

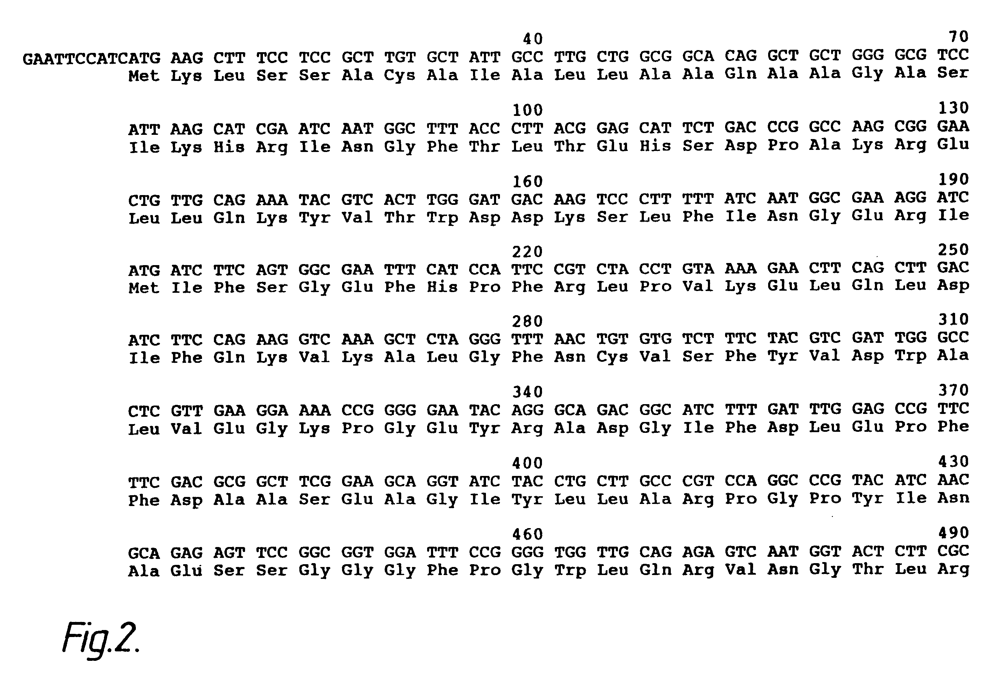

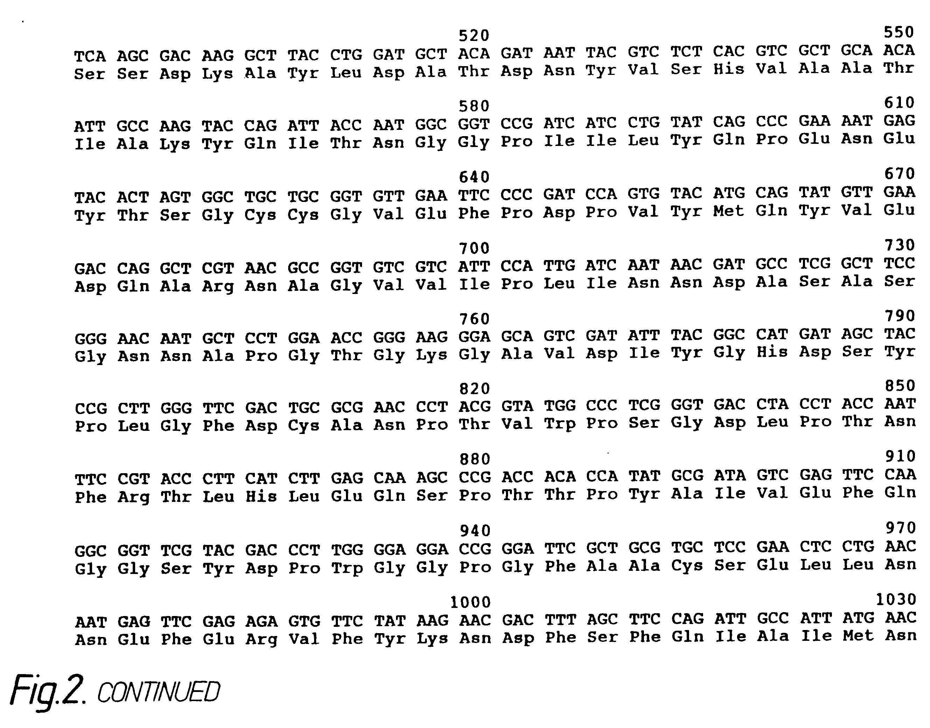

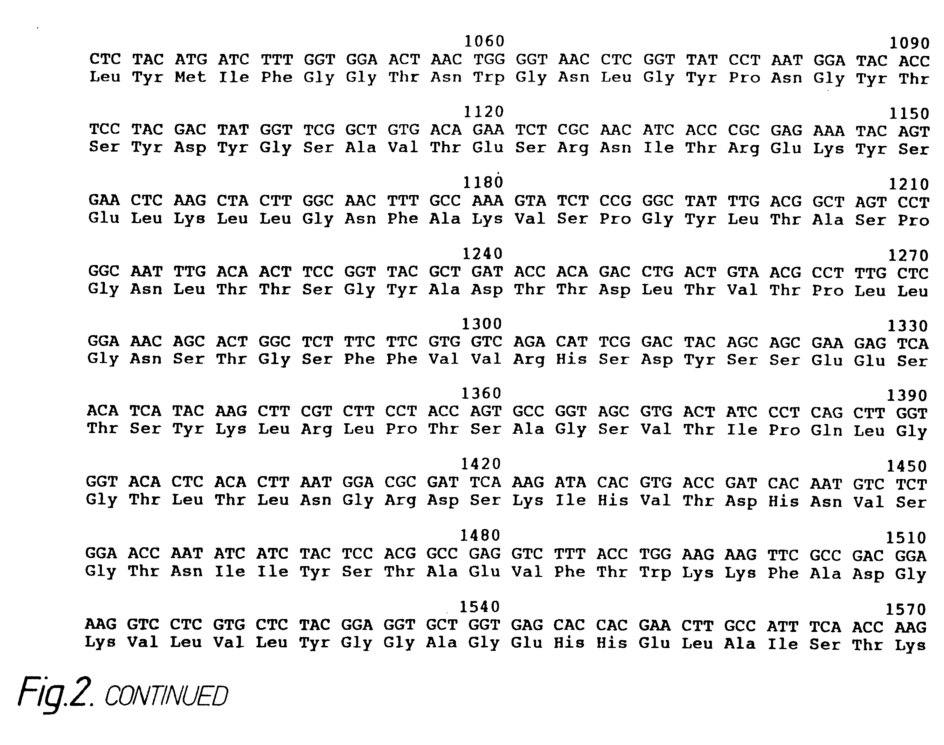

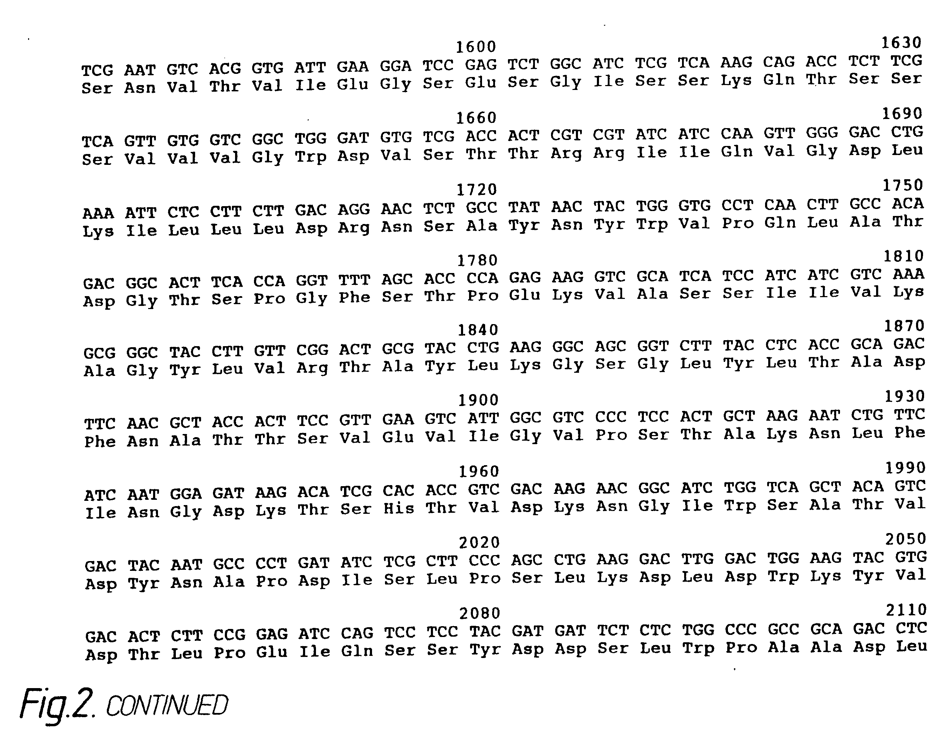

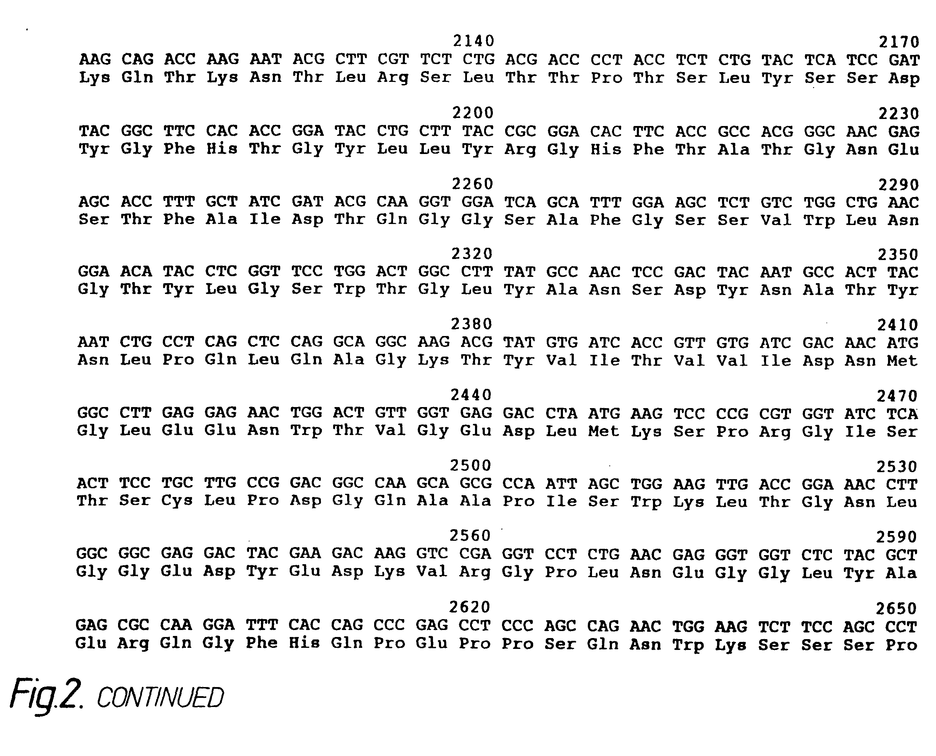

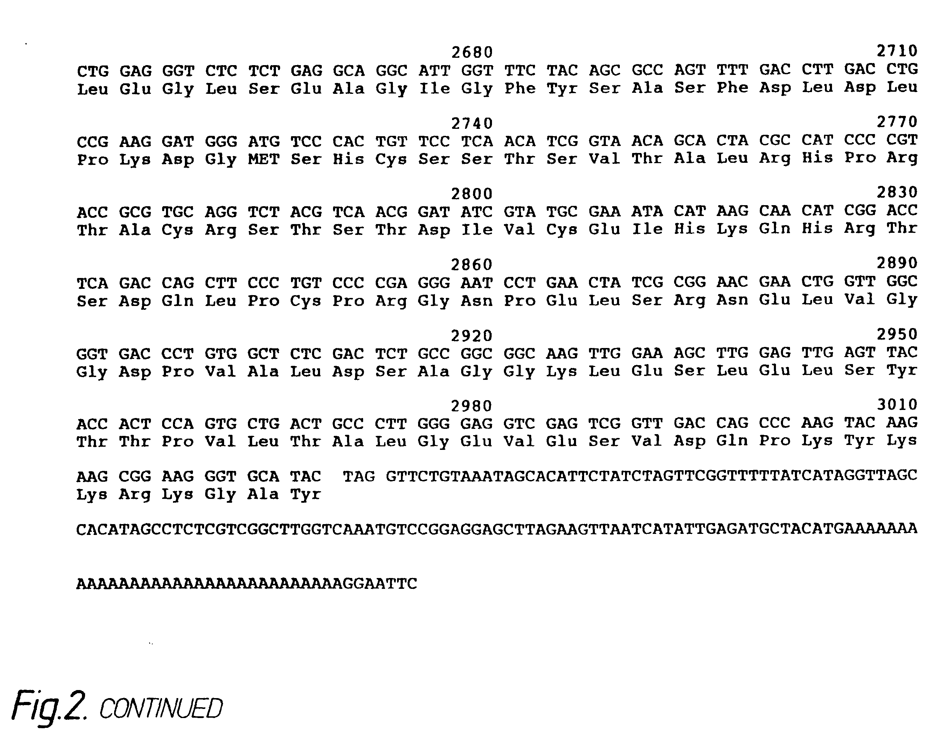

The DNA coding for the β-galactosidase may be from a fungus such as *Aspergillus niger,* preferably from the mutant strain of *Aspergillus niger* designated as VTT-D-80144.. The DNA may be the *Aspergillus niger lacA+* gene and may comprise the sequence from GCC to TAC which is nucleotides 38-3028 of Figure 2.

The yeast may also include the *ADC1* promoter sequence *S*. *cerevisiae.*

The leader sequence may be the sequence ATCG to GCG which is nucleotides 11 to 67 of Figure 2.

The yeast may be deposited under accession no. NCIB 40118 at the National Collection of Industrial and Marine Bacteria (deposited on the 24th February 1989), i.e designated Strain No. DBY 746 (pVK11), containing the plasmid pVK11.

The yeast may be brewers' yeast or a bakers' yeast, such as *S*. *cerevisiae*.

A second aspect of the invention provides a fermentation process in which a yeast according to a first aspect of the invention is grown on a substrate to produce a useful product, wherein the substrate contains lactose for example whey or whey permeate.'.

The useful product may be ethanol, bakers' yeast, a food or nutritional supplement, or silage.

A third aspect of the invention provides a process for the production of a protein, which comprises growing on a substrate containing galactose a second yeast that has been transformed by incorporation of a gene encoding a heterologous protein, other than a secreted β-galactosidase, in which the galactose is produced from lactose by growth in the substrate of a yeast according to a first aspect of the invention, and in which the heterologous protein is subsequently recovered from the substrate.

The second yeast and the yeast according to a first aspect of the invention may be different yeasts, or the same yeasts.

A fourth aspect of the invention provides a process for the production of a protein, which comprises growing on a substrate containing lactose a yeast that has been transformed by incorporation of:
i) a gene encoding a β-galactosidase that is naturally secreted extracellularly; and
ii) a gene encoding a heterologous protein other than a secreted β-galactosidase;
and recovering the heterologous protein from the substrate.

A further aspect of the invention provides a process for the production of a protein, which comprises growing on a substrate containing lactose a mixed flora comprising:
i) a yeast according to a first aspect of the invention; and
ii) a second yeast that has been transformed by incorporation of a gene encoding a heterologous protein, other than a secreted β-galactosidase; and
harvesting the heterologous protein from the resultant broth.

The heterologous protein may be any useful protein. Examples are human serum albumin, glucose isomerase, and detergent enzymes.

As examples of yeasts that can be used in conjunction with a yeast according to the invention in the production of a heterologous protein there can be mentioned the yeasts disclosed in GB-A-2191492 and GB-A-2175590.

An additional utility is envisaged for yeasts such as are described in GB-A-2191492 and GB-A-2175590 that rely for production of heterologous proteins on DNA constructs in which the foreign gene or genes is or are fused to a promoter, such as GAL10, which requires induction by galactose in the medium. In this case such yeasts can be grown upon a conventional medium to high cell density before switching to a medium containing galactose, whereupon the foreign gene is induced. A yeast such as described according to the present invention would allow the second production phase for the heterologous (or foreign) protein to be conducted more cheaply on whey or whey permeate than upon galactose provided externally.

Alternative constructs may be preferred when production of ethanol or of bakers' yeast from whey permeate is the primary target. In that case, excessive secretion of β-galactosidase may be a disadvantage, since the rate of hydrolysis of lactose need not exceed the fermentation rate of the yeast. For many yeasts glucose is a preferred substrate to galactose, so the latter would accumulate in a batch fermentation leading to 'diauxic growth'. For these reasons constructs may be preferred in which the β-galactosidase gene is integrated at a single locus in the yeast chromosome under control of a 'constitutive' yeast promoter. Such constructs can be achieved by 'gene-disruption' techniques that are current art (see for example R.R. Yocum, in Proc. Bioexpo., 1986, Butterworth, Stoneham USA, pp. 171-180).

A suitable host vector into which to insert a DNA construct according to the invention is a yeast such as S. cerevisiae. This organism is suitable for expression and secretion of foreign proteins by recombinant DNA techniques. It may be of advantage to use a cheap fermentation substrate, such as whey or whey permeate in which the components are already food-compatible, in production of such products as therapeutic proteins, vaccines, or industrial enzymes (particularly those involved in food processing). The use for such purposes of yeasts expressing a secreted β-galactosidase is therefore part of this invention. Although in principle many β-galactosidase genes and expression-secretion vectors might be chosen, for various reasons it is preferred to use a gene for a β-galactosidase that is expressed and secreted naturally by a strain of Aspergillus niger, preferably by a mutant strain of Aspergillus niger (VTT-D-80144) (K.M.H. Nevalainen, Appl. Envir. Microbiol., 41. 593, 1981).

One reason is that most reported β-galactosidases are cytoplasmic and hence exist in a highly reducing environment where thiol groups are stable and disulphide bridges are not. Even if they could be secreted across the cytoplasmic membrane, they may not fold correctly or be stable in an oxidising environment.

Another reason is that the requirements for protein secretion are still imperfectly understood. There may be constraints at the levels of primary, secondary or tertiary structure that hinder the secretability of a cytoplasmic protein. This cannot apply to a protein that is naturally secreted.

Amino-terminal 'leader sequences' have been recognised as signals for secretion (see, for example, in Biomembranes. Mansion, L.A. (ed.) 2, 193-195. Plenum Publishing Corp. 1979). These will naturally be present in a pre-protein secreted by Aspergillus and may also serve for secretion in yeast.

A further reason is that both yeast and Aspergillus secrete glycosylated proteins. This is considered important for 'addressing' the proteins to a periplasmic or extracellular location. Also glycosylation may affect enzyme activity. A secreted 'cytoplasmic' enzyme may be aberrantly glycosylated.

The invention is further described in the following Example.

### Example

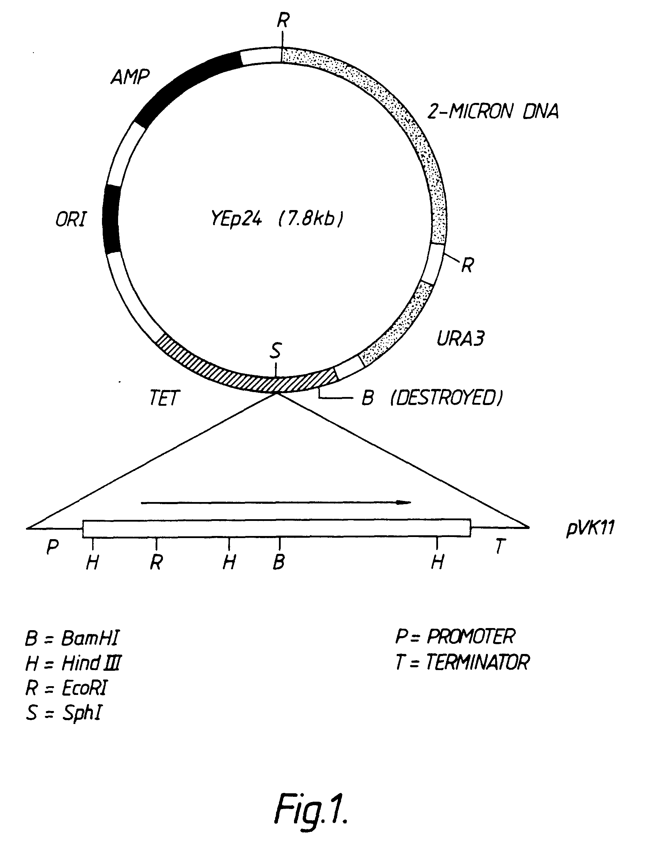

The gene encoding the enzyme β-galactosidase from A.niger VTT-D-80144 was isolated by first making a cDNA library of the fungus using an expression vector λgt11. The construction of cDNA libraries in this way by blunt-end ligation has been described by Tuula T. Teeri, Vijay Kumar, Päivi Lehtovaara, and Jonathan Knowles, Analytical Biochemistry, 164, 60-67 (1987). A cDNA library was preferred to a genomic library in order to circumvent the potential problems of introns being present in the fungal gene; these introns would not be processed by E. coli and probably poorly by S. cerevisiae. The gene was isolated by screening the λgt11 library with antibodies raised against the purified β-galactosidase. Once isolated the gene was then transferred to a yeast plasmid to give the final expression plasmid pVK11. Figure 1 shows the structure of plasmid pVK11 and the restriction map of the 13-galactosidase gene (lacA+) contained in it. This plasmid pVK11 was obtained by inserting the transcription unit consisting of the A. niger β-galactosidase cDNA flanked by the ADC1 promoter at the 5' end and the terminator at the 3' end, into the unique Sph1 site of the yeast plasmid YEp24. The 5' sequence of the gene was determined and is shown in Figure 2; the translation initiation codon and the signal sequence required for secretion are indicated. The boxes indicate the translation initiation points; the solid line indicates the signal sequence and the arrow indicates the putative signal peptidase site.

The plasmid pVK11 was then used to transform S. cerevisiae.

Five criteria were investigated to show that the cloned enzyme is expressed by S. cerevisiae, transformed with pVK11, and is indeed extracellular.

### (1) A histochemical plate assay.

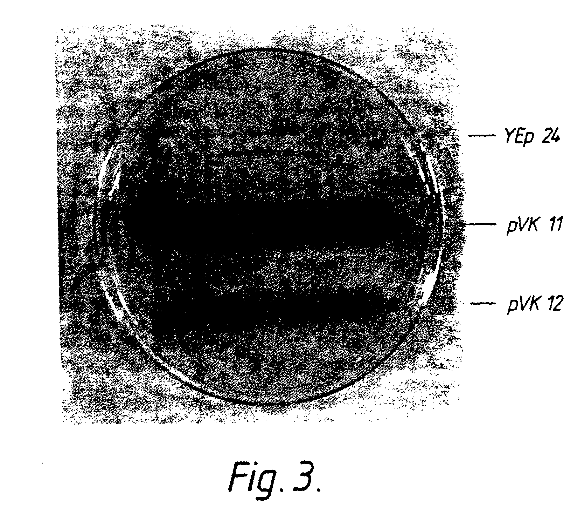

Yeast strains DBY 746, transformed with YEp 24, pVK11 or pVK12 were streaked onto yeast minimal selection plates containing X-gal (40 mg/l). X-gal is a chromogenic substrate of β-galactosidase that produces a blue stain when hydrolysed by the latter. The colour is non-diffusible. pVK12 is a derivative of pVK11 in which ATG₁ bases are deleted so that translation should start at ATG₂ (see Figure 2). The blue halo around DBY 746 (pVK11) visible in Figure 3 is indicative of secretion. The colour reaction for DBY 746 (pVK12) required 4 days incubation at 30°C, compared to overnight for DBY 746 (pVK11), and most of the activity appears intracellular. It will thus be seen that Figure 3 shows that only the cells containing pVK11 show a halo of the X-gal hydrolysis product, indicative of secretion. Non-secreting cells show no such halo. No reaction is seen with normal S. cerevisiae.

### (2) Enzyme assays of sub-cellular fractions.

A yeast culture containing the plasmid pVK11 was centrifuged to separate the cells and the culture medium. The recovered cells were fractionated using standard procedures to separate the cytoplasmic and periplasmic components. β-galactosidase enzyme activity was measured after 50-fold concentration of the separated growth medium, periplasmic and cytoplasmic and total fractions. Table 1 shows that approximately 40% of the β-galactosidase activity is present in the culture medium.

**Table 1**

| Localisation of β-galactosidase produced by yeast strain DBY 746 transformed with plasmid pVK11. | | | | | |
|---|---|---|---|---|---|
| | β-galactosidase | | | ADH | |
| Fraction | Activity units (% total) | | % of total soluble cell protein | Activity units (% total) | |
| Cytoplasmic | 6 | (7%) | - | 51 | (93%) |
| Membrane | 31 | (35%) | - | 2 | (4%) |
| Periplasmic | 17 | (19%) | | 2 | (3%) |
| Intracellular | 54 | (61%) | 0.10 | 55 | (100%) |
| Extracellular | 34 | (39%) | 0.06 | 0 | (0%) |
| TOTAL | 88 | (100%) | 0.16 | 55 | (100%) |

The technique used in obtaining the results set out in Table 1 involved growing to late exponential phase and fractionation according to M.E. Pentilla et al., Yeast, 3, 175, 1988. The reported yields of β-galactosidase are based on specific activity of pure enzyme, 5.35 x 10⁵ units/mg of protein. The distribution of a known cytoplasmic enzyme, alcohol dehydrogenase (ADH), is shown for comparison. Membrane activity was estimated as total intracellular activity less soluble cytoplasmic activity plus periplasmic activity.

### (3) Gel-electrophoresis and immunodetection of secreted β-galactosidase.

Samples of proteins from the growth medium of cells expressing intracellular, extracellular and cells not expressing the β-galactosidase gene were treated with endoglycosidase H. Endoglycosidase H cleaves the sugar moieties from glycoproteins. All secreted yeast proteins are glycosylated. The deglycosylated samples were separated by sodium-dodecyl-sulphate, polyacrylamide gel electrophoresis and then immunoblotted with β-galactosidase specific antibodies. Only cells expressing secreted β-galactosidase showed immunoreactive bands after deglycosylation. Without deglycosylation, the bands appear as a smear of apparent molecular weight greater than the apoprotein of 120 kilodalton subunits, due to variable glycosylation.

### (4) Growth on whey permeate.

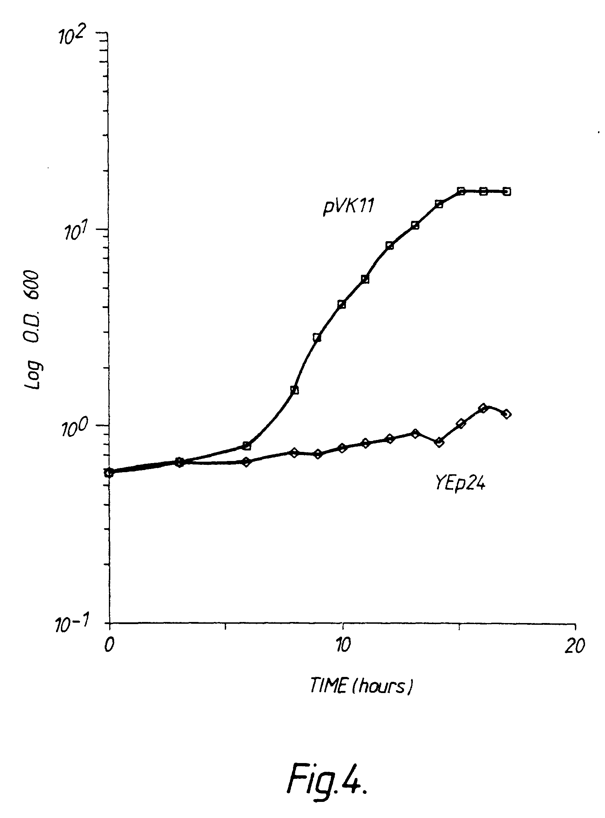

S. cerevisiae cannot grow on lactose because it lacks a lactose uptake mechanism as well as a β-galactosidase activity for lactose hydrolysis. Therefore, in order to grow, the transformants must hydrolyse the lactose extracellularly to a mixture of glucose plus galactose and then transport these into the cell. Figure 4 is a graph of growth on 5% Whey Permeate of S. cerevisiae transformed with YEp24 (◆) or pVK11 (▭) which carried the gene for the A. niger β-galactosidase. It shows that only yeast expressing the cloned β-galactosidase is capable of growth on whey permeate. Hence the relevant enzyme activity must be extracellular and/or periplasmic.

### (5) Lack of diauxic growth on lactose.

Strains of S. cerevisiae normally exhibit diauxic growth in batch culture when grown on a mixture of glucose and galactose. The glucose is used first in a fermentative mode producing ethanol and the galactose is used more slowly, and frequently in respiratory mode which reduces total ethanol yield. In order to demonstrate that yeast transformed with pVK11 does not exhibit diauxic growth a strain of S. cerevisiae GRF167, which grows well on a mixture of galactose and glucose in a minimal medium was transformed with pVK11 as described in this invention. This minimal medium contained per litre: (NH₄)₂SO₄, 20g; MgCl₂, 1.2g; KH₂PO₄, llg; Na-EDTA, 90mg; and trace elements: ZnSO₄, 27mg; MnCl.₄H₂O, 6mg; CoCl₂.6H₂O, 6mg; CuSO₄.5H₂O, 6mg; Na₂MoO₄.2H₂O, 1.8mg; CaCl₂.2H₂O, 2.4mg; FeSO₄.7H₂O, 10mg; H₃BO₃, 6mg; KI 0.6mg; and vitamins added separately after filter sterilization: biotin, 0.03mg; Ca-pantothenate, 0.6mg; nicotinic acid, 0.6mg; myoinositol, 1mg; thiamin-HCl, 0.6mg; pyridoxine-HCl, 6mg.

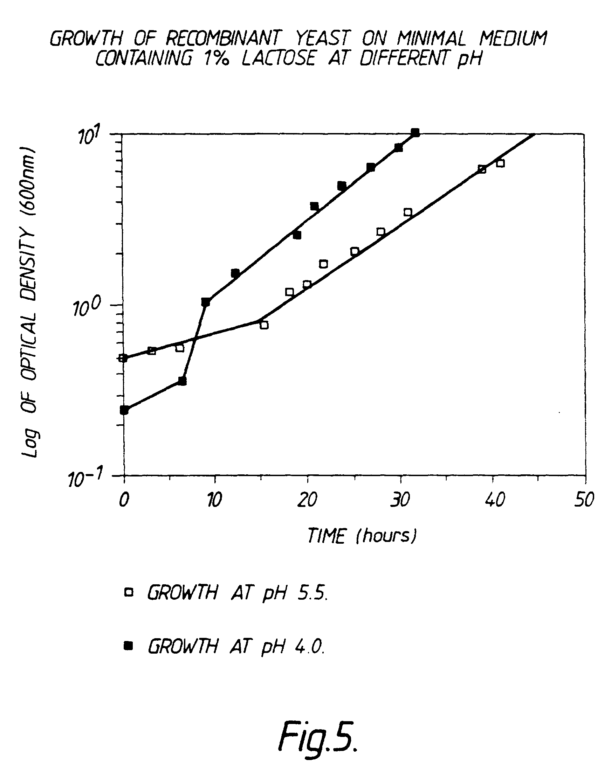

Figure 5 shows the growth rate of this transformant at 30°C on 1% lactose, and in the above minimal medium, at pH 5.5 which is the normal growth optimum of this yeast. However, since the pH optimum of the Aspergillus niger β-galactosidase is pH 4.0 (Widmer and Leuba, Eur. J. Biochem., 100, 559-567, 1979), an exactly similar experiment was conducted at this pH. As can be seen from Figure 5, growth is more rapid at pH 4, and there is no sign of diauxic growth, nor was there any accumulation of glucose or galactose in the medium, indicating that the activity of the secreted β-galactosidase controls the growth rate.

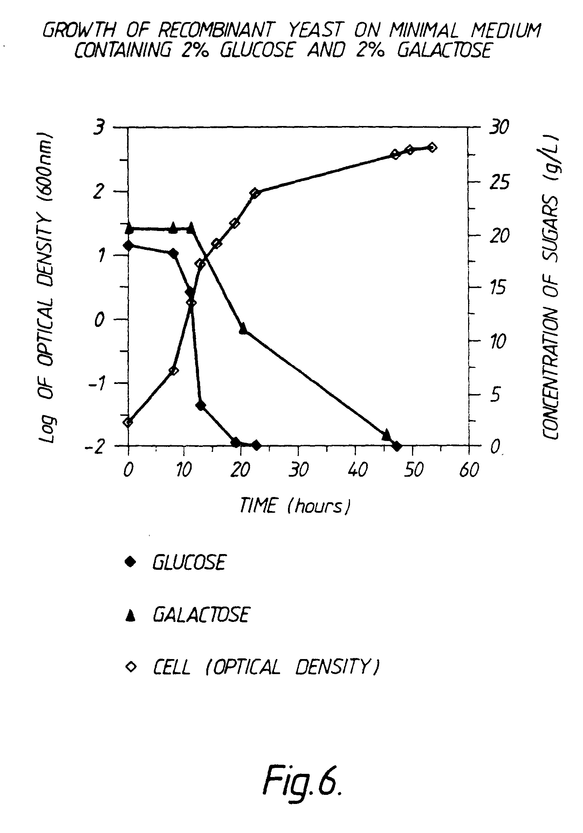

By contrast Figure 6 shows the typical diauxic growth of this yeast on a mixture of glucose and galactose in the same medium at pH 4.0. As expected, it can be seen that growth is rapid on the glucose, which is utilised first, and then slower on the galactose.

## Claims

1. A yeast capable of secreting a protein expressed thereby, the yeast having been transformed with a DNA construct comprising DNA coding for a β-galactosidase, said DNA being obtainable from a source in which the β-galactosidase is naturally secreted extracellularly, said DNA being flanked at its 5' end by a yeast leader sequence, whereby the yeast is capable of growth on lactose as the sole C source.

2. A host according to claim 1, in which the DNA coding for the β-galactosidase is flanked at its 3' end by a terminator sequence.

3. A host according to claim 1 or claim 2, in which the DNA coding for the β-galactosidase is of fungal origin.

4. A host according to claim 3, in which the origin is Aspergillus niger.

5. A host according to claim 4, in which the DNA coding for the β-galactosidase is the Aspergillus niger lacA+ gene.

6. A host according to claim 5, in which the DNA coding for the β-galactosidase comprises the sequence from GCC to TAC which is nucleotides 38-3028 of Figure 2.

7. A host according to any of claims 1 to 6, which comprises the ADC1 promoter sequence of S. cerevisiae.

8. A host according to any of claims 1 to 7, in which the leader sequence is the sequence from ATG to GCG which is nucleotides 11-67 of Figure 2.

9. A yeast as deposited on 24th February 1989 at The National Collection of Industrial and Marine Bacteria, under Accession No. NCIB 40118.

10. A host according to any of claims 1 to 8, which is a brewers' yeast or a bakers' yeast.

11. A host according to any of claims 1 to 8, which is of the species Saccharomyces cerevisiae.

12. A fermentation process in which a yeast is grown on a substrate to produce a useful product, in which the yeast is as defined in any of claims 1 to 11 and in which the substrate contains lactose.

13. A process according to claim 12, in which the useful product is ethanol.

14. A process according to claim 12, in which the useful product is bakers' yeast.

15. A process according to claim 12, in which the useful product is a food or a nutritional supplement.

16. A process according to claim 12, in which the useful product is silage.

17. A process for the production of a protein, which comprises growing on a substrate containing galactose a second yeast that has been transformed by incorporation of a gene encoding a heterologous protein, other than a secreted β-galactosidase, in which the galactose is produced from lactose by growth in the substrate of a yeast according to any of claims 1 to 11, and in which the heterologous protein is subsequently recovered from the substrate.

18. A process according to claim 17, in which the second yeast and the yeast according to any of claims 1 to 11 are different yeasts.

19. A process according to claim 17, in which the second yeast is also a yeast according to any of claims 1 to 11.

20. A process for the production of a protein, which comprises growing on a substrate containing lactose a yeast that has been transformed by incorporation of (i) a gene encoding a β-galactosidase that is naturally secreted extracellularly and (ii) a gene encoding a heterologous protein other than a secreted β-galactosidase, and recovering the heterologous protein from the substrate.

21. A process for the production of a protein, which comprises growing on a substrate containing lactose a mixed flora comprising (i) a yeast according to any of claims 1 to 11 and (ii) a second yeast as defined in claim 17, and harvesting the heterologous protein from the resultant broth.

## Patentansprüche

1. Hefe, die befähigt ist, ein durch sie exprimiertes Protein zu sekretieren, wobei die Hefe mit einem DNA-Konstrukt transformiert wurde, das DNA umfaßt, die für eine β-Galactosidase codiert, wobei die DNA aus einer Quelle erhältlich ist, in der die β-Galactosidase natürlicherweise extrazellulär sekretiert wird, und wobei die DNA an ihrem 5'-Ende von einer Hefe-Leader-Sequenz flankiert wird, wodurch die Hefe zum Wachstum auf Lactose als einziger C-Quelle befähigt ist.

2. Wirt nach Anspruch 1, worin die für die β-Galactosidase codierende DNA an ihrem 3'-Ende von einer Terminatorsequenz flankiert wird.

3. Wirt nach Anspruch 1 oder Anspruch 2, worin die Herkunft der für die β-Galactosidase codierenden DNA ein Pilz ist.

4. Wirt nach Anspruch 3, worin die Herkunft Aspergillus niger ist.

5. Wirt nach Anspruch 4, worin die für die β-Galactosidase codierende DNA das lacA⁺-Gen von Aspergillus niger ist.

6. Wirt nach Anspruch 5, worin die für die β-Galactosidase codierende DNA die Sequenz von GCC bis TAC entsprechend den Nukleotiden 38-3028 von Figur 2 umfaßt.

7. Wirt nach irgendeinem der Ansprüche 1 bis 6, welcher die ADC1-Promotorsequenz von S. cerevisiae umfaßt.

8. Wirt nach irgendeinem der Ansprüche 1 bis 7, worin die Leader-Sequenz die Sequenz von ATG bis GCG entsprechend den Nukleotiden 11-67 von Figur 2 umfaßt.

9. Hefe, wie am 24. Februar 1989 bei "The National Collection of Industrial and Marine Bacteria" unter der Zugangs-Nr. NCIB 40118 hinterlegt.

10. Wirt nach irgendeinem der Ansprüche 1 bis 8, der eine Brauhefe oder eine Bäckerhefe ist.

11. Wirt nach irgendeinem der Ansprüche 1 bis 8 der Spezies Saccharomyces cerevisiae.

12. Fermentationsverfahren, worin eine Hefe zur Herstellung eines zweckmäßigen Produkts auf einem Substrat wachsen gelassen wird, worin die Hefe wie in irgendeinem der Ansprüche 1 bis 11 definiert ist und worin das Substrat Lactose enthält.

13. Verfahren nach Anspruch 12, worin das zweckmäßige Produkt Ethanol ist.

14. Verfahren nach Anspruch 12, worin das zweckmäßige Produkt Bäckerhefe ist.

15. Verfahren nach Anspruch 12, worin das zweckmäßige Produkt ein Nahrungs- oder Nahrungsergänzungsmittel ist.

16. Verfahren nach Anspruch 12, worin das zweckmäßige Produkt Silage ist.

17. Verfahren zur Herstellung eines Proteins, welches das Wachsenlassen, auf einem Galactose enthaltenden Substrat, einer zweiten Hefe umfaßt, die durch Einbau eines für ein heterologes Protein codierenden Gens, das von der sekretierten β-Galactosidase verschieden ist, transformiert wurde, worin die Galactose durch Wachstum einer Hefe nach irgendeinem der Ansprüche 1 bis 11 in dem Substrat aus Lactose produziert wird und worin das heterologe Protein anschließend aus dem Substrat gewonnen wird.

18. Verfahren nach Anspruch 17, worin die zweite Hefe und die Hefe nach irgendeinem der Ansprüche 1 bis 11 unterschiedliche Hefen sind.

19. Verfahren nach Anspruch 17, worin die zweite Hefe ebenfalls eine Hefe nach irgendeinem der Ansprüche 1 bis 11 ist.

20. Verfahren zur Herstellung eines Proteins, umfassend das Wachsenlassen einer Hefe, die durch Einbau (i) eines Gens, das für eine β-Galactosidase codiert; die natürlicherweise extrazellulär sekretiert wird, und (ii) eines Gens, das für ein heterologes Protein codiert, das von einer sekretierten β-Galactosidase verschieden ist, transformiert wurde, auf einem Lactose enthaltenden Substrat und Gewinnung des heterologen Proteins aus dem Substrat.

21. Verfahren zur Herstellung eines Proteins, umfassend das Wachsenlassen einer Mischflora, die (i) eine Hefe nach irgendeinem der Ansprüche 1 bis 11 und (ii) eine zweite, wie in Anspruch 17 definierte Hefe umfaßt, auf einem Lactose enthaltenden Substrat und Ernten des heterologen Proteins aus dem resultierenden Nährmedium.

## Revendications

1. Levure capable de sécréter une protéine exprimée par elle, la levure ayant été transformée avec un produit de recombinaison d'ADN comprenant la codification de l'ADN pour une β-galactosidase, cet ADN étant susceptible d'être obtenu à partir d'une source dans laquelle la β-galactosidase est naturellement sécrétée à l'extérieur de la cellule, cet ADN étant accolé à son extrémité 5' d'une séquence initiale de levure, la levure étant capable de croître sur le lactose comme source unique de C.

2. Hôte selon la revendication 1, dans lequel l'ADN codant pour la β-galactosidase est accolé à son extrémité 3' d'une séquence terminateur.

3. Hôte selon la revendication 1 ou la revendication 2,
dans lequel
l'ADN codant pour la β-galactosidase est d'origine fongique.

4. Hôte selon la revendication 3,
dans lequel
l'origine est Aspergillus niger.

5. Hôte selon la revendication 4, dans lequel la codification de l'ADN de la β-galactosidase est le gène lacA+ de Aspergillus niger.

6. Hôte selon la revendication 5, dans lequel l'ADN codant pour la β-galactosidase comprend la séquence allant de GCC à TAC qui est représentée par les nucléotides 38-3028 de la figure 2.

7. Hôte selon l'une quelconque des revendications 1 à 6, qui comprend la séquence du promoteur ADC1 de S. cerevisiae.

8. Hôte selon l'une quelconque des revendications 1 à 7,
dans lequel
la séquence initiale est la séquence allant de ARG à GCG qui est représentée par les nucléotides 11-67 de la figure 2.

9. Levure comme déposée le 24 février 1989 à la collection nationale de bactérie industrielle et marine, sous le numéro d'accession No. NCIB 40118.

10. Hôte selon l'une quelconque des revendications 1 à 8,
qui est
une levure de brasseurs ou une levure de boulanger.

11. Hôte selon l'une quelconque des revendications 1 à 8,
qui provient des espèces Saccharomyces cerevisiae.

12. Procédé de fermentation dans lequel une levure se développe sur un substrat pour produire un produit utile,
dans lequel
la levure est comme définie selon l'une quelconque des revendications 1 à 11 et dans lequel le substrat contient du lactose.

13. Procédé selon la revendication 12,
dans lequel
le produit utile est l'éthanol.

14. Procédé selon la revendication 12,
dans lequel
le produit utile est la levure de boulanger.

15. Procédé selon la revendication 12,
dans lequel
le produit utile est un supplément alimentaire ou nutritionnel.

16. Procédé selon la revendication 12,
dans lequel
le produit utile est de l'ensilage.

17. Procédé pour la production d'une protéine,
qui comprend
la croissance sur un substrat contenant du galactose d'une deuxième levure qui a été transformée par incorporation d'un gène codant pour une protéine hétérologue, autre qu'une β-galactosidase sécrétée, dans laquelle le galactose est produit à partir de lactose par croissance dans le substrat de la levure selon l'une quelconque des revendications 1 à 11, et dans laquelle la protéine hétérologue est ensuite récupérée du substrat.

18. Procédé selon la revendication 17,
dans lequel
la deuxième levure et la levure selon l'une quelconque des revendications 1 à 11 sont des levures différentes.

19. Procédé selon la revendication 17,
dans lequel
la deuxième levure est aussi une levure selon l'une quelconque des revendications 1 à 11.

20. Procédé pour la production d'une protéine,
qui comprend
- la croissance sur un substrat contenant du lactose d'une levure qui a été transformée par incorporation de
(i) un gène codant pour une β-galactosidase qui est naturellement sécrétée à l'extérieur de la cellule et
(ii) un gène codant pour une protéine hétérologue autre qu'une β-galactosidase sécrétée, et
- la récupération de la protéine hétérologue du substrat.

21. Procédé pour la production d'une protéine, qui comprend :
- la croissance sur un substrat contenant du lactose d'une flore mixte comprenant
(i) une levure selon l'une quelconque des revendications 1 à 11 et
(ii) une deuxième levure telle que définie dans la revendication 17, et
- la récupération de la protéine hétérologue du bouillon résultant.
